**Europäisches Patentamt**

**European Patent Office**

**Office européen des brevets**

(19)

(11) Numéro de publication : **0 463 923 B1**

(12) ## FASCICULE DE BREVET EUROPEEN

(45) Date de publication du fascicule du brevet :
**16.03.94 Bulletin 94/11**

(51) Int. Cl.[5] : **C07C 17/156,** C07C 19/045,
B01J 23/72, B01J 8/32

(21) Numéro de dépôt : **91401608.4**

(22) Date de dépôt : **17.06.91**

(54) **Procédé et catalyseur d'oxychloration; leur application à la production du 1,2-dichloroéthane.**

(30) Priorité : **25.06.90 FR 9007932**

(43) Date de publication de la demande :
**02.01.92 Bulletin 92/01**

(45) Mention de la délivrance du brevet :
**16.03.94 Bulletin 94/11**

(84) Etats contractants désignés :
**AT BE CH DE DK ES FR GB GR IT LI LU NL SE**

(56) Documents cités :
**EP-A- 0 278 922
EP-A- 0 377 364
DE-A- 1 468 073
FR-A- 1 359 016
FR-A- 2 067 306
FR-A- 2 242 143**

(73) Titulaire : **ELF ATOCHEM S.A.
4 & 8, Cours Michelet La Défense 10
F-92800 Puteaux (FR)**

(72) Inventeur : **Petit, Alain, Villa Lou Miradou
Ancienne Route de Marseille
F-13500 Martigues (FR)**
Inventeur : **Bachelard, Roland
99 Grande Rue de la Guillotière
F-69007 Lyon (FR)**
Inventeur : **Clair, René, La Vrignoise
Saint-Julien
F-13500 Martigues (FR)**
Inventeur : **Correia, Yves
Les Lauzières
F-04160 Château-Arnoux (FR)**

## Description

La présente invention concerne un procédé et un catalyseur d'oxychloration et leur application à la production du 1,2 dichloroéthane. Le 1,2-dichloroéthane (D 12) est un produit fabriqué industriellement à raison de plusieurs millions de tonnes par an, qui par pyrolyse donne du chlorure de vinyle monomère (VCM) et de l'acide chlorhydrique (HCl). Le VCM est polymérisé en poly(chlorure de vinyle) (ou PVC) matière plastique couramment utilisée. L'HCl obtenu à la pyrolyse est séparé du VCM puis est mis en contact avec de l'éthylène et de l'oxygène en présence d'un catalyseur pour donner du D 12, c'est la réaction d'oxychloration. Cette réaction est très générale et peut être effectuée avec la plupart des hydrocarbures. L'oxychloration a été décrite dans de nombreux brevets, en particulier dans FR 2 063 365, FR 2 260 551, FR 2 213 259 et FR 2 125 748. Le catalyseur est constitué d'un sel de cuivre déposé sur de l'alumine en poudre. La demande de brevet européen EP 58 644 décrit la préparation d'un catalyseur effectuée en versant une solution de chlorure cuivrique dans un lit fluidisé de poudre d'alumine et à une température maximum de 50°C. Cette opération est suivie d'un séchage à l'air chaud en lit fluide et sans dépasser 140°C. La demande de brevet européen EP 29 143 décrit la préparation d'un catalyseur d'oxychloration consistant à mélanger dans un lit fluide en réaction d'oxychloration du catalyseur déjà préparé, c'est-à-dire de la poudre d'alumine contenant un composé du cuivre et du support nu c'est-à-dire de la poudre d'alumine ne contenant pas de composé de cuivre. On décrit aussi l'addition du support nu à un lit fluide en réaction d'oxychloration primitivement chargé avec du catalyseur. Cette méthode est présentée comme ayant l'avantage d'éviter le collage (ou agglomération) des grains de catalyseur pendant le fonctionnement. Cette demande décrit une migration du composé de cuivre depuis l'alumine contenant le cuivre vers le support nu. Il est bien clair que le résultat de cette méthode est une réduction de la teneur en cuivre du catalyseur puisqu'on augmente la quantité de support sans ajouter de cuivre. Cette demande présente bien le fait que le résultat : "Suppression ou réduction du collage" n'est pas lié seulement à la réduction de la quantité de cuivre mais aussi à la migration d'un composé de cuivre depuis l'alumine contenant le composé de cuivre vers le support nu. Cette demande EP 29 143 décrit aussi un moyen de préparer un catalyseur i n situ en chargeant le réacteur avec du support nu, le fluidisant avec les gaz de réaction et y ajoutant du chlorure cuivrique solide. Ce moyen est présenté comme un substitut à la préparation conventionnelle du catalyseur par imprégnation en dehors du réacteur. Cependant cette préparation n'est suggérée qu'à l'échelle du laboratoire, en effet ce mode de préparation n'est pas industriel puisqu'au début de cette préparation le lit fluide ne contient pas de catalyseur proprement dit, c'est-à-dire un composé de cuivre sur un support, il n'y a donc pas de réaction d'oxychloration, il est nécessaire de chauffer, et si on chauffe alors qu'il n'existe pas de catalyseur on se trouve en sortie du lit fluide dans une zone explosive puisqu'il n'y a pas réaction, donc pas de conversion.

La demande de brevet EP 119933 décrit aussi un catalyseur d'oxychloration selon le même principe que précédemment, c'est-à-dire du cuivre imprégnant une poudre d'alumine mais n'ayant pas l'inconvénient de coller, parce-que, dit-on, dans cette demande il y a moins de cuivre à la surface qu'à l'intérieur du grain de catalyseur.

Tout cet art antérieur concerne des lits fluides d'oxychloration utilisant un catalyseur "homogène", c'est-à-dire des poudres dont tous les grains sont imprégnés de cuivre. Il existe aussi des lits fluides d'oxychloration utilisant un catalyseur "hétérogène", c'est-à-dire des poudres constituées d'un mélange de grains d'alumine imprégnés de cuivre comme précédemment et de grains inertes tels que du sable siliceux. Ces catalyseurs sont décrits dans le brevet FR 2 242 143. Ces catalyseurs "hétérogènes" ne présentent pas l'inconvénient du collage des catalyseurs "homogènes" mais par contre ont l'inconvénient de former un mélange auto-abrasif, les grains de sable provoquent l'usure des grains d'alumine. Les fines riches en cuivre sont éliminées et il faut compenser par des appoints de catalyseur neuf.

On a maintenant trouvé un perfectionnement à ces catalyseurs "hétérogènes" d'oxychloration qui permet de maintenir ses performances constantes dans le temps.

Pour éviter des confusions avec les termes habituels de catalyse homogène et catalyse hétérogène et rester cohérents avec les termes de FR 2 242 143, les catalyseurs "hétérogènes" d'oxychloration seront appelés : charge fluidisable, ou charge catalytique fluidisable.

La présente invention est donc un procédé d'oxychloration d'un hydrocarbure pour former un hydrocarbure chloré dans lequel l'hydrocarbure, un gaz contenant de l'oxygène et de l'acide chlorhydrique gazeux passent sur une charge fluidisable comprenant un mélange d'un catalyseur d'oxychloration et de particules d'au moins une substance solide catalytiquement et chimiquement inerte caractérisée en ce qu'on ajoute dans la charge fluidisée une solution ou une suspension d'un composé de cuivre.

La présente invention concerne aussi ces compositions de catalyseur.

L'invention est donc une composition utilisable comme catalyseur d'oxychloration caractérisée en ce qu'elle comprend un mélange d'un catalyseur d'oxychloration et de particules d'au moins une substance solide catalytiquement et chimiquement inerte dans lequel on a ajouté une solution ou une suspension d'un composé de cuivre.

Dans le procédé selon EP-A-377 364, pertinent au titre de la nouveauté seulement, c'est du cuivre ou un composé de cuivre qui est ajouté dans la charge fluidisée.

L'hydrocarbure peut être un mélange de plusieurs hydrocarbures, choisis parmi les hydrocarbures aliphatiques en $C_1$ à $C_{20}$, les cycloaliphatiques jusqu'en $C_{12}$ et les aromatiques ayant jusqu'à 4 noyaux benzéniques condensés ainsi que leurs dérivés de substitution chlorés. Ils peuvent être choisis par exemple parmi le méthane, l'éthane, le propane, l'éthylène et le propylène. L'invention est particulièrement utile pour l'éthylène. Le gaz contenant de l'oxygène est tout simplement de l'air mais on peut aussi l'utiliser, appauvri ou enrichi en oxygène.

Les catalyseurs d'oxychloration utilisables dans l'invention peuvent être tout catalyseur d'oxychloration utilisables en eux-mêmes sans mélange avec une substance catalytiquement et chimiquement inerte. Avantageusement on utilise des poudres essentiellement à base d'alumine de granulométrie entre 20 et 200 $\mu$ et de surface entre 90 et 450 m²/g et de préférence entre 30 et 90 $\mu$ et entre 250 et 400 m²/g. Ces poudres sont imprégnées de cuivre ou d'un sel de cuivre en quantité pouvant aller jusqu'à 10 %, et de préférence 3 à 10 % en poids de cuivre par rapport au catalyseur fini.

Comme substance catalytiquement et chimiquement inerte jouant le rôle de diluant (mais en aucun cas de support de catalyseur), on peut citer, notamment, les micro-billes de verre ou de silice, l'alumine alpha et, de préférence, le sable siliceux que l'on trouve à l'état naturel et dont la répartition granulométrique de la taille des particules est adaptée aux besoins de la fluidisation.

La répartition granulométrique du catalyseur proprement dit, d'une part, et celle de la substance catalytiquement et chimiquement inerte, d'autre part, sont choisies de telle façon que le diamètre et l'étalement de la taille des particules du mélange soient propices à une bonne fluidisation.

Avantageusement là taille des particules inertes est comprise entre 20 et 200 $\mu$.

La quantité de substance inerte peut varier dans de larges limites. Avantageusement la quantité d'inerte peut présenter de 1 à 20 fois en poids la quantité de catalyseur.

Le but de l'oxychloration est essentiellement d'utiliser l'acide chlorhydrique comme source de chlore. On ajuste donc la quantité d'oxygène et d'hydrocarbure pour obtenir à peu près stoechiométriquement l'hydrocarbure chloré en consommant le maximum d'HCl et d'hydrocarbure.

Au cours du fonctionnement d'un lit fluidisé d'oxychloration on constate une baisse d'activité se traduisant par un manque de conversion de l'hydrocarbure.

Cette baisse est due à une usure du catalyseur. A cette usure qui entraine une baisse d'activité s'ajoute une perte physique par un entrainement par les gaz en sortie du lit fluide et une efficacité imparfaite des moyens de séparation entre le catalyseur entrainé et les gaz. Cette séparation est rendue difficile par la transformation d'une partie du catalyseur en poussière du fait de l'attrition. Généralement on compense la réduction de la quantité de catalyseur et la diminution de son activité par des ajouts de catalyseur neuf. La demanderesse a constaté avec surprise qu'on pouvait compenser cette baisse d'activité en ajoutant dans le lit fluide une solution ou une suspension d'un composé de cuivre.

Parmi les composés de cuivre on peut utiliser le chlorure, l'oxychlorure. On préfère utiliser le chlorure cuivrique.

La solution peut être une suspension si sa concentration est élevée ou si le composé de cuivre est peu soluble.

La solution du composé de cuivre peut être une solution aqueuse. Cette solution ou suspension s'ajoute dans le réacteur d'oxychloration en continu ou discontinu mais pendant le fonctionnement de l'oxychloration. On peut l'introduire par une buse de pulvérisation.

La quantité de cuivre à introduire sous forme de solution est fonction des performances qu'on recherche. L'opération peut être renouvelée autant de fols que nécessaire. On peut aussi en plus de cette solution d'un composé de cuivre ajouter du cuivre en poudre , un composé de cuivre en poudre ou du catalyseur neuf contenant du cuivre.

Le cuivre ou un composé de cuivre en poudre peut être sous forme de catalyseur très riche en cuivre. Très riche en cuivre veut dire que sa teneur exprimée en cuivre en pourcentage poids du catalyseur fini est supérieure à celle du catalyseur en fonctionnement dans la charge fluidisée. Avantageusement cette valeur est 1,2 fois et de préférence comprise entre 1,5 et 3 fois celle du catalyseur en fonctionnement dans la charge fluidisée.

Par exemple si le catalyseur en fonctionnement dans la charge fluidisée contient entre 3,5 et 7 % en poids de cuivre, on fait des ajouts de catalyseur à environ 12 % en poids de cuivre. On considère que cette teneur de 12 % exprimée en cuivre est très riche en cuivre. De plus, c'est une valeur inhabituelle parce que le procédé pour l'obtenir est beaucoup plus compliqué que pour les valeurs usuelles de 3 à 8 %.

L'invention concerne aussi des compositions catalytiques ou des charges fluidisables utilisées dans le procédé de la présente invention qu'on vient de décrire.

3

L'avantage de l'invention par rapport au fonctionnement traditionnel qui consiste à compenser la baisse d'activité et la diminution de la quantité de catalyseur seulement par des ajouts de catalyseur neuf, est une marche plus régulière, on évite les à-coups d'activité, et l'amélioration est plus durable.

Les exemples suivants illustrent l'invention.

## EXEMPLE 1

1/ Essai n° 1 : Référence des conditions normales de marche (non conforme à l'invention).

Dans un réacteur de 3 m de diamètre, produisant 25 tonnes/h de dichloro-1,2 éthane, les conditions opératoires sont les suivantes :

Température     : 245 à 250°C
Pression        : 4 bar
Temps de séjour : 25 à 30 s

Système catalytique : 8 tonnes d'alumine imprégnée à 6 % de cuivre (catalyseur) plus silice (22 tonnes). L'alumine a une surface de 357 m²/g, un diamètre moyen de 53 $\mu$, un volume poreux de 33 cm²/100 g et une masse volumique tassée de 1192 Kg/m³. La silice est un sable de Fontainebleau (silice pure) de diamètre moyen 50 $\mu$ et compris entre 20 et 300 $\mu$.

Voir les résultats obtenus dans le tableau I ci-après.

2/ Essai n° 2 conforme à l'invention : Avec ajouts d'une solution de chlorure cuivrique

Mêmes conditions que pour l'essai n° 1 avec, en plus, ajouts d'une solution de chlorure cuivrique. On fait des ajouts discontinus correspondants à des quantités de cuivre métal de 50 à 200 kg. Les résultats sont donnés dans le tableau I suivant :

EP 0 463 923 B1

TABLEAU I

| | $\dfrac{HCl}{C_2H_4}$ (mole/mole) | $\dfrac{O_2}{C_2H_4}$ (mole/mole) | $Y_G$ (%) Taux de conversion de l'HCl | $X_{D12}$ (%) Taux de conversion en D12 | $R\ C_2H_4$ (%) Taux de conversion en chlorés | EVENT (% volume) | | | | Consommation Cata (g/t D12) | Cu % pds du lit | Consommation Cu (g/t D12) |
|---|---|---|---|---|---|---|---|---|---|---|---|---|
| | | | | | | $C_2H_4$ | $CO_2$ | CO | $O_2$ | | | |
| Essai I | 1,97 | 0,68 | 99,5 | 95,5 | 96,8 | 0,42 | 1,20 | 0,60 | 5 | 250 | 1 à 1,2 | 0 |
| Essai II | 1,99 | 0,63 | 99 | 96 | 97,3 | 0,30 | 1,00 | 0,65 | 4,25 | 120 | 2,5 à 3 | 25 |

- La consommation en catalyseur traduit les ajouts de catalyseur identiques au catalyseur formant le système catalytique initial (catalyseur + silice)

- La consommation de cuivre est une consommation de chlorure cuivrique en solution exprimée en cuivre.

On a indiqué dans le tableau I la consommation de catalyseur et la consommation de solution de chlorure cuivrique exprimées en grammes par tonne de D 12 fabriqué. Les additions de catalyseur et de solution de chlorure cuivrique sont faites pour obtenir des conditions opératoires constantes.

**Revendications**

1. Procédé d'oxychloration d'un hydrocarbure pour former un hydrocarbure chloré dans lequel l'hydrocarbure, un gaz contenant de l'oxygène et de l'acide chlorhydrique gazeux passent sur une charge fluidisable comprenant un mélange d'un catalyseur d'oxychloration et de particules d'au moins une substance solide catalytiquement et chimiquement inerte, caractérisé en ce qu'on ajoute dans la charge fluidisée une solution ou une suspension d'un composé de cuivre.

2. Procédé selon la revendication 1 caractérisé en ce que l'hydrocarbure est de l'éthylène.

3. Procédé selon la revendication 1 ou 2 caractérisé en ce que la quantité de particules inertes représente de 1 à 20 fois en poids la quantité de catalyseur.

4. Procédé selon l'une des revendications 1 à 3 caractérisé en ce que le composé de cuivre est du chlorure cuivrique.

5. Procédé selon l'une des revendications 1 à 4 caractérisé en ce qu'on ajoute aussi du cuivre en poudre ou un composé de cuivre en poudre.

6. Charge fluidisable utilisable dans un réacteur d'oxychloration caractérisée en ce qu'elle comprend un mélange (i) d'un catalyseur d'oxychloration et (ii) des particules d'au moins une substance solide catalytiquement et chimiquement inerte dans lequel on a ajouté une solution ou une suspension d'un composé de cuivre.

7. Charge fluidisable selon la revendication 6 caractérisée en ce que c'est la charge utilisée dans le procédé des revendications 1 à 5.

**Claims**

1. Process for the oxychlorination of a hydrocarbon to form a chlorinated hydrocarbon, in which the hydrocarbon, an oxygen-containing gas and gaseous hydrochloric acid are passed through a fluidizable charge consisting of a mixture of an oxychlorination catalyst and particles of at least one catalytically and chemically inert solid substance, characterized in that a solution or a suspension of a copper compound is added to the fluidized charge.

2. Process according to Claim 1, characterized in that the hydrocarbon is ethylene.

3. Process according to Claim 1 or 2, characterized in that the quantity of inert particles is 1 to 20 times the weight of the quantity of catalyst.

4. Process according to one of Claims 1 to 3, characterized in that the copper compound is copper chloride.

5. Process according to one of Claims 1 to 4, characterized in that copper as a powder or a copper compound as a powder is also added.

6. Fluidizable charge which can be used in an oxychlorination reactor, characterized in that it consists of a mixture of (i) an oxychlorination catalyst and (ii) particles of at least one catalytically and chemically inert solid substance to which a solution or a suspension of a copper compound has been added.

7. Fluidizable charge according to Claim 6, characterized in that it is the charge used in the process of Claims 1 to 5.

## Patentansprüche

1.  Verfahren zur Oxichlorierung eines Kohlenwasserstoffes unter Bildung eines chlorierten Kohlenwasserstoffes, in dem dieser Kohlenwasserstoff und ein Gas, welches Sauerstoff und gasförmigen Chlorwasserstoff enthält, über einen fluidisierenden Füllstoff geführt werden, welcher eine Mischung aus einem Oxichlorierungskatalysator und Partikeln wenigstens einer festen katalytisch und chemisch inerten Substanz enthält, dadurch gekennzeichnet, daß man dem fluidisierenden Füllstoff eine Lösung oder eine Suspension einer Kupferverbindung hinzufügt.

2.  Verfahren nach Anspruch 1, dadurch gekennzeichnet, daß der Kohlenwasserstoff Ethylen ist.

3.  Verfahren nach Anspruch 1 oder 2, dadurch gekennzeichnet, daß die Menge der inerten Partikel das 1- bis 20-fache des Gewichts der Menge an Katalysator beträgt.

4.  Verfahren nach einem der Ansprüche 1 bis 3, dadurch gekennzeichnet, daß die Kupferverbindung das Kupfer(II)-chlorid ist.

5.  Verfahren nach einem der Ansprüche 1 bis 4, dadurch gekennzeichnet, daß man weiterhin pulverförmiges Kupfer oder eine Kupferverbindung in Pulverform hinzufügt.

6.  Fluidisierbarer Füllstoff, verwendbar in einem Oxichlorierungsreaktor, dadurch gekennzeichnet, daß er eine Mischung aus
    (i) einem Oxichlorierungskatalysators und
    (ii) Partikeln wenigstens einer festen katalytisch und chemisch inerten Substanz
    enthält, zu der man eine Lösung oder eine Suspension einer Kupferzusammensetzung hinzufügt,

7.  Fluidisierbarer Füllstoff nach Anspruch 6, dadurch gekennzeichnet, daß dieser Füllstoff im Verfahren gemäß den Ansprüchen 1 bis 5 verwendet wird.